# EUROPEAN PATENT APPLICATION

(11) **EP 2 131 196 A1**
(43) Date of publication of application: **09.12.2009**
(21) Application number: 08157478.2
(22) Date of filing: 03.06.2008
(51) Int. Cl.: G01N 33/543

(54) **Detection of analytes on a flow-through biosensor**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

The present invention relates to flow-through membranes and biosensors suitable for the immobilization and rapid detection of analytes, methods for the preparation of such membranes and the use of such membranes for the immobilization of biomolecules and subsequent detection of immobilized biomolecules.

## Description

### FIELD OF THE INVENTION

Subject of the present invention is a flow-through biosensor suitable for the immobilization and rapid detection of analytes, methods for the preparation of membranes for such biosensors and the use of such biosensors for the immobilization of biomolecules and subsequent detection of immobilized biomolecules.

### BACKGROUND OF THE INVENTION

The immobilization of biomolecules is a prerequisite in many standard assays for the detection of analytes, e.g. biomolecules. Such assays are used in a variety of fields such as molecular biology, immunology, cell biology, biochemistry in clinical situations, where these assays serve in diagnostics and also in food science. Receptor molecules that are able to specifically bind to analytes, with high affinity are immobilized on a substrate, e.g. a membrane, and then contacted with a solution in order to detect analytes in that solution. Upon binding to their receptor, analytes (e.g. biomolecules) are detected, e.g. by the use of specific probes. Most commonly used detection systems are optically and based on fluorescence, refractive index changes or spectral changes. For example, the fluorescence of the bound analytes itself or of fluorescently labeled probes is detected. For high-throughput screening applications, a high number of receptor molecules that specifically bind to different analytes have to be present on one substrate. Most often biochips, gene chips or other microarrays are used to which the receptor molecules have been attached.

Biomolecules detected in standard immobilization assays include DNA, RNA, proteins, cells, small molecules, toxins and drugs present within a sample. Accordingly, the receptor molecules ("capture probes") most often used are oligonucleotides, DNA, RNA or antibodies to which the biomolecules to be detected ("capture targets") hybridize to (in case of nucleic acids and oligonucleotides) or bind to (in case of e.g. proteins).

In point-of-care diagnostics, immobilization and detection of biomolecules is performed using dedicated devices, e.g. small bench top analyzers (for example, blood gas and electrolyte systems) and hand held, single use devices (such as urine albumin, blood glucose, and coagulation tests). In point-of-care diagnostics, rapid and accurate generation of test results is desired so that an appropriate treatment can be implemented, leading to an improved clinical or economic outcome. Even with the most sophisticated device, reliable results can be obtained only if the patient is prepared appropriately and the correct technique is used. As point-of-care testing is likely to be done by staff with limited technical background, training and quality control are critical.

Flow-through and lateral flow membranes and devices are for example used in the detection of analytes, e.g. biomolecules in samples. This applies particularly for point-of-care tests, where biological samples such as body fluids, e.g. blood or urine, are tested for the presence of specific analytes.

### SUMMARY OF THE INVENTION

The present invention relates to a membrane for the immobilization of biomolecules on spots on said membrane, wherein a capture probe is immobilized on a spot on said membrane and a detection probe is immobilized on the backside of the membrane on said spot and wherein capture probe and detection probe may bind to the same biomolecule.

Preferably, the membrane of the present invention is a flow-through membrane.

Subject of the present invention is also an assay comprising a membrane of the present invention. Preferably, the assay is a flow-through assay.

The present invention also relates to a device comprising the membrane of the invention. Said device is preferably a device for the immobilization and subsequent detection of a biomolecule in a sample.

Subject of the present invention is also a method for making the membrane according to the present invention, wherein the membrane is a flow-through membrane, comprising the steps of:
- attaching a capture probe to one side of the membrane, and
- attaching a detection probe to the opposite side of the membrane,
wherein the capture probe and the detection probe are allowed to dry on said membrane.

Also within the scope of the present invention is a method of detecting a biomolecule from a sample comprising the steps of:
- providing the membrane according to the present invention,
- bringing the sample into contact with a capture probe spot on said membrane under conditions allowing for the binding of the biomolecule to be detected to the capture probe, thereby immobilizing the biomolecule to be detected,
- applying a flow directed from the backside of the membrane where a detection probe is immobilized on a spot to the foreside of the membrane where a capture probe is immobilized on said spot on said membrane, thereby transferring the detection probe to the foreside of the membrane under conditions allowing for the binding of the detection probe to the biomolecule to be detected,
- detecting a detection probe bound to an immobilized biomolecule on said spot on the foreside of the membrane.

The present invention in addition relates to the use of any of the membranes, methods, assays and devices according to the present invention for the immobilization and detection of biomolecules.

### BRIEF DESCRIPTION OF THE DRAWINGS

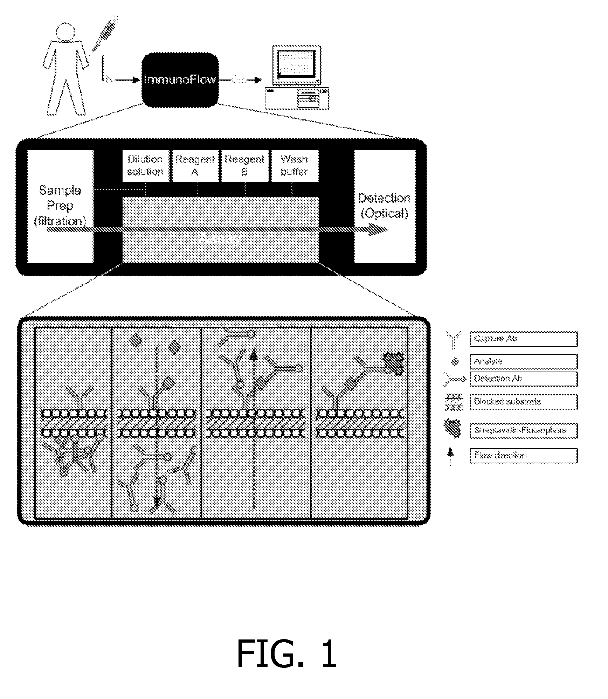

Figure 1: Illustration of an exemplary setup of a device comprising a membrane according to the present invention and illustration of the method of immobilizing and detecting biomolecules on such a membrane.

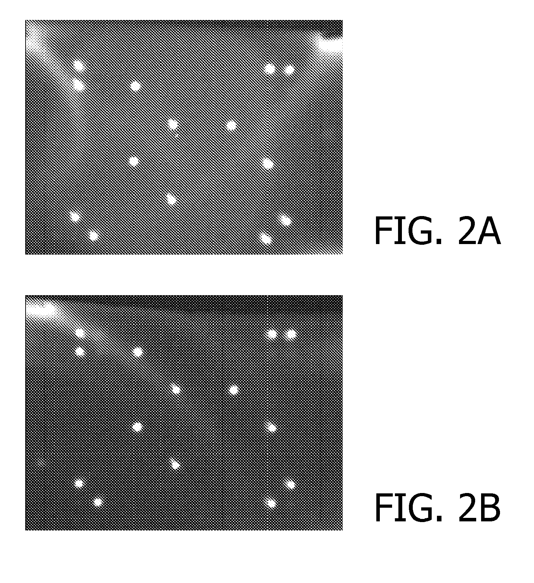

Figure 2: Visualization of the detection antibody with a fluorescent label after printing (A) and after incubation for several minutes with fluorescently labeled streptavidin (B).
Figure 3: Comparison of the method of immobilization and detection according to the present invention using detection antibodies printed on the backside of the membrane (left graphs, PI) to a conventional flow-through assay (right, II) with two different concentrations (left bars of each assay: 1 nM; right bar of each assay: 1 pM) of biomolecule to be detected.
Figure 4: Illustrative schematic print layout of antibodies deposited on the nitrocellulose membrane of example 1. 1: corner markers; 2: Donkey anti-sheep AF633 antibody (internal calibrators to normalize the signal intensity, 1 µM); 3: Mouse anti-CRP antibody (8µM); 4: array markers

### DETAILED DESCRIPTION OF EMBODIMENTS

The present invention relates to a membrane for the immobilization of biomolecules on spots on said membrane, wherein a capture probe is immobilized on a spot on said membrane and a detection probe is immobilized on the backside of the membrane on said spot and wherein capture probe and detection probe may bind to the same biomolecule.

A capture probe or a detection probe may in the context of this invention preferably be pluralities of capture probes and pluralities of detection probes, respectively. According to the present invention a "spot" is a well-defined area of the membrane. Capture probes are attached to the membrane and immobilized in said spots.

In preferred embodiments of the invention the membrane comprises functional groups in said spots for the attachment of capture probes.

In particular embodiments of the invention, said functional groups are selected from the group consisting of vinyl, epoxide, thiol, aldehydes, carboxyl, hydroxyl, amine, succimide and lysine groups. Particularly, said capture probes are covalently attached to said functional groups. A preferred way of covalently coupling is via the standard NHS/EDC chemistry which is known to a person skilled in the art. According to the present invention it is however not necessary to couple capture probes covalently. It is also possible that immobilizing of capture probes as achieved by physical adsorption via hydrophobic interactions; e.g. in case of antibodies.

In other particular embodiments of the invention said functional groups are selected from the group consisting of amides, peptides, hydroxyl or other groups where proteins non-covalently bind. Particularly, in this case said capture probes are non-covalently attached to said functional groups.

Preferably, the membrane of the present invention is a flow-through membrane.

The membrane may preferably selected from the group, comprising nylon, nitrocellulose and Poly(ether sulfone) (PES).

The membrane may be a flow-through membrane and have pores, preferably the pores may have sizes in the range of from about 0.45 µm to 5 µm. The thickness of the membrane may preferably be in the range of from about 50 µm to 250µm.

The capture probe may in preferred embodiments be selected from the group comprising oligonucleotides, DNA, RNA, aptamers, amplicons, antibodies or functional fragments of antibodies. Most preferably, the capture probe is an antibody or a functional fragment thereof.

The detection probe may preferably be selected from the group comprising oligonucleotides, DNA, RNA, aptamers, amplicons, antibodies or functional fragments of antibodies. Most preferably, the detection probe is an antibody or a functional fragment thereof. The detection probe may further comprise additional components such as compounds selected from the group comprising enzymes and oligonucleotides.

In a particular preferred embodiment, the capture probe and/or the detection probe are in a dry format, i.e. the attached probes are dried on the membrane. Dried probes on the membrane ("dry format") e.g. facilitates the use of such a membrane in a (disposable) cartridge.

In some embodiments, the capture probe is covalently or non-covalently attached to said spots of the membrane.

Preferably, the detection probe is attached to said spots of the membrane by ink jet printing or deposited using other contact methods, e.g. by pipetting. Most preferably, the detection probe is allowed to dry after application to the backside of the membrane.

Thus in a preferred embodiment of the membrane, the capture probe is covalently or non-covalently attached to said spots of the membrane and wherein the capture probe is selected from the group comprising oligonucleotides, DNA, RNA, aptamers, amplicons, antibodies or functional fragments of antibodies.

It is further preferred that the detection probe is attached to said spots of the membrane by ink jet printing or deposited using other contact methods and wherein the detection probe is selected from the group comprising oligonucleotides, DNA, RNA, aptamers, amplicons, antibodies or functional fragments of antibodies.

The membrane may in a particular embodiment be a membrane for the immobilization of more than one biomolecule, wherein more than one capture probe is immobilized on a spot on said membrane and more than one detection probe is immobilized on the backside of the membrane wherein a capture probe and a detection probe immobilized on the same spot may bind to the same biomolecule.

Subject of the present invention is also an assay comprising a membrane of the present invention. Preferably, the assay is a flow-through assay. In such an assay, biomolecules to be detected are, if present in the sample, immobilized on the foreside of the membrane via capture probes by bringing the sample suspected of containing such biomolecules in contact with the foreside of the membrane. This may for example be done by a lateral flow over the membrane or by a flow through the membrane. After this contacting step, a flow from the backside of the membrane through the membrane is applied such that detection probes are transferred to the foreside of the membrane. There, the detection probes are allowed to bind to the immobilized and capture-probe-bound biomolecules to be detected. The binding of the detection probes to the immobilized biomolecules is then detected after an optional washing step for washing away unbound and unspecifically bound biomolecules and detection probes. The detection can either be a direct detection, e.g. when the detection probe is labeled, e.g. with a fluorescent dye or a gold particle or indirectly via a label (e.g. a streptavidin-fluorophore complex binding to a biotinylated detection probe, e.g. an biotinylated antibody) binding to the detection probe, wherein said label can be detected.

Also within the scope of the present invention is a cartridge comprising the membrane of the present invention. The cartridge may preferably be a disposable cartridge. The membrane of the present invention may in a particular embodiment be comprised in a disposable cartridge.

The present invention also relates to a device comprising the membrane of the invention. Said device is preferably a device for the immobilization and subsequent detection of a biomolecule in a sample.

Preferably, the device additionally comprises a filter for filtering the sample before contacting the sample with said membrane and/or a detector for detecting membrane-bound biomolecules or membrane-bound labels. The optional filter of the device may serve for the preparation of samples, e.g. whole blood or urine, by filtering out components of the sample that are not to be contacted with the capture probes, detection probes or membrane.

The detector may preferably be an optical detector for the detection of the detection probes bound to biomolecules to be detected, wherein the biomolecules to be detected are immobilized on the membrane via capture probes. Most preferably, the optical detector is capable to detect fluorescence of the bound detection probes or of fluorophores directly or indirectly (e.g. via avidin or streptavidin) bound to the detection probe.

In one embodiment of the device of the present invention said membrane is comprised in a disposable cartridge and the device further comprises a receiving unit for receiving said cartridge.

Most preferably, the device of the present invention may be used for the detection of the presence of specific biomolecules in a sample by applying the sample to the device, applying the method or assay of the present invention and retrieving a results regarding to the presence or non-presence of the specific biomolecule in the sample.

Subject of the present invention is also a method for making the membrane according to the present invention, wherein the membrane is a flow-through membrane, comprising the steps of:
- attaching a capture probe to one side of the membrane, and
- attaching a detection probe to the opposite side of the membrane,
wherein the capture probe and the detection probe are allowed to dry on said membrane.

In some embodiments, additional reagents may be added to the side of the membrane where the detection probes are attached. Such reagents may preferably be assay diluents in a dry format.

The method of making said membrane may comprise a step for the addition of capture probes to spots on said membrane. Inkjet printing is a preferred method according to the present invention. Other means can also be applied as hand pipetting or complex offset printing.

The basis of the membrane itself may be a commercially available membrane which is modified according to the present invention by the attachment of capture probes and/or detection probes.

Also within the scope of the present invention is a method of detecting a biomolecule from a sample comprising the steps of:
- providing the membrane according to the present invention,
- bringing the sample into contact with a capture probe spot on said membrane under conditions allowing for the binding of the biomolecule to be detected to the capture probe, thereby immobilizing the biomolecule to be detected,
- applying a flow directed from the backside of the membrane where a detection probe is immobilized on a spot to the foreside of the membrane where a capture probe is immobilized on said spot on said membrane, thereby transferring the detection probe to the foreside of the membrane under conditions allowing for the binding of the detection probe to the biomolecule to be detected,
- detecting a detection probe bound to an immobilized biomolecule on said spot on the foreside of the membrane.

In some embodiments, the method of detecting a biomolecule from a sample additionally comprises the step of:
- washing away unbound detection probe from the foreside of the membrane before detecting said detection probe.

The method of detecting a biomolecule from a sample may also in a particular embodiment additionally comprise the step of:
- bringing a label which may bind to the detection probe into contact with the foreside of the membrane under conditions allowing for the binding of said label to said detection probe,
wherein detecting the detection probe comprises detection of the label bound to said detection probe.

A particular embodiment of the invention relates to a method of detecting a biomolecule from a sample, wherein the detection probe is biotinylated and the label is a streptavidin-fluorophore and wherein fluorescence of said streptavidin-fluorophore is detected.

In particularly preferred embodiment of the method, the presence of one or more specific biomolecules in a sample is detected. The sample may for example be blood or parts thereof, such as serum or plasma, or other body fluids such as urine, lymph and the like.

The membranes, devices, methods and assays of the present invention may be used for quantitative or qualitative analysis of samples for the presence of one or more specific biomolecules to be detected. The signal detected from the biomolecule-bound detection probes may be analyzed qualitatively or quantitatively, e.g. this signal may be proportional to the concentration of biomolecule to the detected in the sample.

The present invention in addition relates to the use of any of the membranes, methods, assays and devices according to the present invention for the immobilization and detection of biomolecules.

In particular uses of the membranes, methods of detecting, assays, cartridge and devices according to the present invention, the presence of one or more specific biomolecules in a sample is detected.

Particularly, the biomolecules are selected from the group comprising proteins, peptides, oligonucleotides, RNA, DNA, antibodies, tissues, cells, drugs and chemical compounds.

Preferably the use is a use for ELISA, bioassays, binding and interaction assays or nucleic acid hybridization assays.

Preferably, the membranes according to this invention are used for the detection of biomolecules bound to said capture probes.

The biomolecules bound to said capture probes are for example detected by fluorescently labeled probes. Other labels may be chemiluminescent, enzymatic, or conductivity labels.

For example the membranes according to the present invention may be used for ELISA assays, bioassays, binding and interaction assays, nucleic acid hybridization assays.

"Biomolecules" in the context of this invention are molecules naturally occurring in living organisms or molecules that are otherwise biologically relevant or molecules derived therefrom. Particularly relevant biomolecules in the context of this invention are macromolecules such as peptides, proteins, oligosaccharides, oligonucleotides and nucleic acids, like DNA, RNA, LNA and PNA.

"Oligonucleotides" in the context of this invention are short sequences of nucleotides with 2 to 200 nucleotides, particularly sequences with 2 to 20 nucleotides.

Functional groups in the context of this invention are groups on the surface of the membrane spots to which capture probes can be attached, e.g. covalently attached or non-covalently attached. In case of a covalent attachment of the capture probes, the functional groups may be for example acrylate, epoxide, thiol, carboxyl, hydroxyl and amine groups. Other groups are described above.

The term "antibody" in the context of the present invention comprises monoclonal and polyclonal antibodies and binding fragments thereof, in particular Fc-fragments as well as so called "single-chain-antibodies" (Bird R. E. et al (1988) Science 242:423-6), chimeric, humanized, in particular CDR-grafted antibodies, and dia- or tetrabodies (Holliger P. et al (1993) Proc. Natl. Acad. Sci. U.S.A. 90:6444-8) and "lama antibodies". Also comprised are immunoglobulin like proteins that are selected through techniques including, for example, phage display to specifically bind to the polypeptides of the present invention.

### EXAMPLES

### Example 1: Illustrative experimental set-up

C-Reactive protein (CRP) was used as an assay model. A CRP sandwich assay was performed. CRP capture probes (8 µM) 27,droplets were printed in triplicate on the nitrocellulose membrane (front side). Also printed were Donkey anti-sheep AF 633 (1 µM) as internal calibrators and corner markers to lay the grid. The nitrocellulose membranes were blocked for 60 min using 5% BSA+ PBS (500µl/well) blocking buffer. This blocking is performed to minimize binding of the analyte, detection antibody and Streptavidin to the membrane. The CRP analyte was pumped for 5 min on the ImmunoFlow protein platform.

Subsequently different incubation steps were used:
print detection antibody, print streptavidin
print detection antibody, incubate streptavidin
incubate detection antibody, incubate streptavidin

All steps were performed in duplo for 2 concentrations: 1 nM and 1 pM. Only for the printed detection + printed streptavidin a blank was taken.

| *Experiment* | *Concentration analyte* | *Detection antibody* | *Streptavidin* |
|---|---|---|---|
| PP1P | 1 pM | Printed | Printed |
| PI1P | 1 pM | Printed | Incubated |
| II1P | 1 pM | Incubated | Incubated |
| PP1N | 1 nM | Printed | Printed |
| PI1N | 1 nM | Printed | Incubated |
| II1N | 1 nM | Incubated | Incubated |

Detection antibodies and streptavidin were deposited on the opposite side of the arrays. This was done by pipetting the required amount of material and allowing drying for 5 minutes. Due to the relative large amount of solution pipetted (6.94 µl/array detection antibody and 5 µl/array Streptavidin) the membranes were completely wet. This resulted in fluid going through the membrane and some of it was deposited on the bottom of the wells plate. Therefore the concentrations used may differ from the wanted concentration. After drying, the arrays were put in a new well.

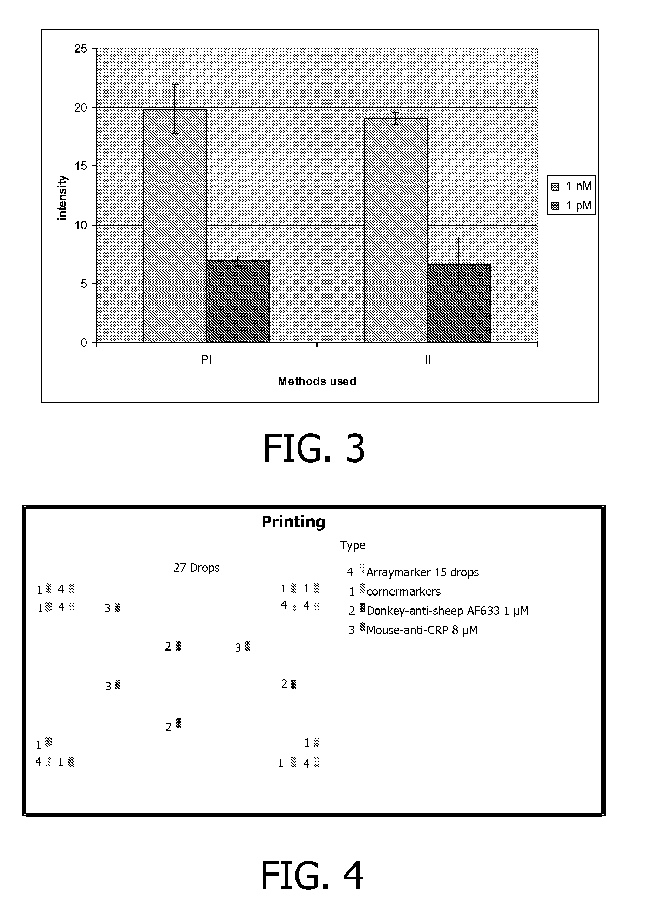

After every incubation step, the arrays were washed (PBS + 0.05% Tween) 3 times 5 min the washing buffer. Finally, the arrays were dried and analyzed using the LED-setup. The results are shown in appended Fig. 3.

## Claims

1. A membrane for the immobilization of biomolecules on spots on said membrane, wherein a capture probe is immobilized on a spot on said membrane and a detection probe is immobilized on the backside of the membrane on said spot and wherein capture probe and detection probe may bind to the same biomolecule.

2. A membrane according to claim 2, wherein the membrane is a flow-through membrane.

3. A membrane according to claims 1 or 2, wherein the capture probe is covalently or non-covalently attached to said spots of the membrane and wherein the capture probe is selected from the group comprising oligonucleotides, DNA, RNA, aptamers, amplicons, antibodies or functional fragments of antibodies.

4. A membrane according to claims 1 to 3, wherein the detection probe is attached to said spots of the membrane by ink jet printing or deposited using other contact methods and wherein the detection probe is selected from the group comprising oligonucleotides, DNA, RNA, aptamers, amplicons, antibodies or functional fragments of antibodies.

5. A membrane according to claims 1 to 4, wherein the capture probe and/or the detection probe are in a dry format.

6. A membrane according to claims 1 to 5 for the immobilization of more than one biomolecule, wherein more than one capture probe is immobilized on a spot on said membrane and more than one detection probe is immobilized on the backside of the membrane wherein a capture probe and a detection probe immobilized on the same spot may bind to the same biomolecule.

7. A flow-through assay comprising a membrane according to claims 1 to 6.

8. A disposable cartridge comprising a membrane according to claims 1 to 6.

9. A device comprising a membrane according to claims 1 to 6.

10. Device according to claim 9, additionally comprising a filter for filtering the sample before contacting the sample with said membrane and/or a detector for detecting membrane-bound biomolecules or membrane-bound labels.

11. A method for making a membrane according to claims 1 to 6, wherein the membrane is a flow-through membrane, comprising the steps of:
- attaching a capture probe to one side of the membrane, and
- attaching a detection probe to the opposite side of the membrane,
wherein the capture probe and the detection probe are allowed to dry on said membrane.

12. A method of detecting a biomolecule from a sample comprising the steps of:
- providing a membrane according to claims 1 to 6;
- bringing the sample into contact with a capture probe spot on said membrane under conditions allowing for the binding of the biomolecule to be detected to the capture probe, thereby immobilizing the biomolecule to be detected;
- applying a flow directed from the backside of the membrane where a detection probe is immobilized on a spot to the foreside of the membrane where a capture probe is immobilized on said spot on said membrane, thereby transferring the detection probe to the foreside of the membrane under conditions allowing for the binding of the detection probe to the biomolecule to be detected;
- detecting a detection probe bound to an immobilized biomolecule on said spot on the foreside of the membrane.

13. A method according to claim 12, additionally comprising the step of:
- bringing a label which may bind to the detection probe into contact with the foreside of the membrane under conditions allowing for the binding of said label to said detection probe,
wherein detecting the detection probe comprises detection of the label bound to said detection probe.

14. The use of any of the membranes according to claims 1 to 6 or the assay of claim 7 or of the cartridge of claim 8 or of any of the devices of claims 10 or 11 or of any of the methods of claims 12 to 13 for the immobilization and detection of biomolecules.

15. The use according to claim 14 for ELISA, bioassays, binding and interaction assays or nucleic acid hybridization assays.
